# EUROPEAN PATENT APPLICATION

(11) **EP 4 369 020 A1**
(43) Date of publication of application: **15.05.2024**
(21) Application number: 22206031.1
(22) Date of filing: 08.11.2022
(51) Int. Cl.: G01R 33/36

(54) **RADIO FREQUENCY ANTENNA SYSTEM**

(71) Applicant: Koninklijke Philips N.V., 5656 AG Eindhoven (NL)
(72) Inventor: LEUSSLER, Christoph Günther, Eindhoven (NL); FINDEKLEE, Christian, Eindhoven (NL); VERNICKEL, Peter, 5656AG Eindhoven (NL)
(74) Representative: Philips Intellectual Property & Standards

(57) **Abstract**

According to the invention, a radio frequency antenna system (1) for a magnetic resonance imaging device comprising a radio frequency coil (2) which, when mounted in a bore of the magnetic resonance imaging device has a total coil impedance composed of a coil impedance and a patient-specific impedance of a patient, a preamplifier (3) connected to the radio frequency coil (2), by means of which a signal received by the radio frequency coil (2) can be amplified and an amplified output signal can be output, a matching network (4) which is interconnected between the radio frequency coil (2) and the preamplifier (3) with which the total coil impedance of the radio frequency coil (2) is adjustable and a controller (5), wherein the radio frequency coil (2) comprises a first coil loop (6) and at least one further coil loop (7), the controller (5) is connected to the radio frequency coil (2), the matching network (4) and the preamplifier (3) and the controller (5) is adapted for noise impedance matching between the radio frequency coil (2) and the preamplifier (3) as a function of patient-specific parameters by controlling the employment of the first coil loop (6) and the at least one further coil loop (7) and by controlling the matching network (4). In this way, a radio frequency antenna system with improved impedance matching is provided.

## Description

### FIELD OF THE INVENTION

The invention relates to the field of magnetic resonance imaging. In particular, the present invention relates to the field of radio frequency antenna systems for a magnetic resonance imaging device.

### BACKGROUND OF THE INVENTION

A part of every magnetic resonance imaging device is the radio frequency antenna system for receiving and converting the magnetic resonance signal. The radio frequency antenna system comprises a radio frequency coil, a matching network, a preamplifier and an analog-to-digital converter. In magnetic resonance imaging a system integrated body coil generates the excitation magnetic field for the spin system causing a preceding relaxation. The precession of the net magnetization induces a current in the radio frequency coil via electromagnetic induction. The radio frequency coil comprises generally a conducting wire with an inductance and a coil resistance. Like any radio frequency operated system, the radio frequency antenna system is subject to noise. Thereby, the most important requirement is to achieve a minimum noise figure. The lowest noise figure is achieved if the source impedance is matched to the noise impedance of the preamplifier. Especially, for magnetic resonance imaging in the low field range (< 1 T), the signal-to-noise ratio depends on the patient geometry, the size of the bore and the relative position of the radio frequency coil within the bore. This means that the signal-to-noise ratio cannot be maintained at a constant level, as it depends on the patient, the coil to be used and the position of the coil on the patient.

The article 'An adjustable RF coil loading device' by C.E. Hayes in Magnetic Resonance Imaging, 99(1993) 81-86; describes an adjustable loading device having two conducting end rings connected by an adjustable, even number of uniformly spaced resistive straight segments or strips. This adjustable loading device can substitute for the tissue losses of various sized patients in a whole-body magnetic resonance imaging device. The conductor structure is not changed with respect to loading. The birdcage structure consists of fixed resistors. The whole setup mimics a liquid load phantom within a transmit/receive coil.

Accurate and efficient impedance matching is critical in magnetic resonance imaging to achieve a minimum noise figure. However, most of the existing methods suggest radio frequency coils used for an imaging entity incapable of on-sight patient and device dependent matching.

### SUMMARY OF THE INVENTION

It is the object of the invention to provide a radio frequency antenna system with improved impedance matching.

According to the invention, this object is addressed by the subject matter of the independent claims. Preferred embodiments of the invention are described in the dependent claims.

Therefore, according to the invention, a radio frequency antenna system for a magnetic resonance imaging device is provided, comprising a radio frequency coil which, when attached to a patient has a total coil impedance composed of a coil impedance and a patient-specific impedance, a preamplifier connected to the radio frequency coil, by means of which a signal received by the radio frequency coil can be amplified and an amplified output signal can be output, a matching network which is interconnected between the radio frequency coil and the preamplifier with which the total coil impedance of the radio frequency coil is adjustable and a controller, wherein the radio frequency coil comprises a first coil loop and at least one further coil loop, the controller is connected to the radio frequency coil, the matching network and the preamplifier and the controller is adapted for noise impedance matching between the radio frequency coil and the preamplifier as a function of patient-specific parameters by controlling the employment of the first coil loop and the at least one further coil loop and by controlling the matching network.

Here, the term "attached to a patient" means that the radio frequency coil is located with respect to the patient in a bore of the magnetic resonance imaging device. Advantageously, the radio frequency coil may be placed on the patient's body surface to reduce signal transmission through air. However, as well known by the person skilled in the art, this is not necessarily the case. In the case of a birdcage coil or the like, it is not possible for the radio frequency coil to be arranged on the surface of the patient body, rather in this case the radio frequency coil is in the vicinity of the patient but still near to the patient in the bore of the magnetic resonance imaging device. The bore is an opening in the magnetic resonance imaging device designed to accommodate a treatment table with a patient and a radio frequency coil.

As stated before, the radio frequency coil generally comprises a piece of a conductive wire with an inductance and a coil resistance which, if loaded with a patient, changes to the total coil impedance which is the combination of the radio frequency coil impedance and the patient's impedance.

This must be taken into account when the received radio signal is passed on to the preamplifier, which has an impedance which is different from the total coil impedance. For low-noise signal transmission, patient-specific impedance matching is therefore recommended in order to adapt the total coil impedance to the impedance of the preamplifier. The objective is to achieve a minimum noise figure, which is defined as the ratio of an input signal-to-noise ratio to an output signal-to-noise ratio. The output signal-to-noise ratio corresponds to the output signal-to-noise ratio of the preamplifier.

Especially, magnetic resonance imaging low field systems below 1 T need a reconsideration of the radio frequency coil topology. While radio frequency coils at higher field strengths are mainly patient loaded, radio frequency coils at lower field strengths can easily show additional electronic noise contributions if the coils are not well designed. Moreover, in clinical application radio frequency coils are directly placed on the patient body or dedicated coils are applied, which fit the shape of the patient body. However, there are clinical situations, where the optimal coil to tissue distance cannot be applied such as for pediatric imaging, magnetic resonance employed in linear accelerators and interventional applications.

Thus, in the case of low field magnetic resonance imaging the total coil impedance, and by that the noise behavior, may not be dominated by inductive loading of the patient's body but radio frequency coil design itself. Simultaneously, additional total coil impedance adjustments must be taken into account for stretchable or flexible radio frequency coils due to the variation in shape of the radio frequency coil.

This is accounted for by having the controller connected to both the radio frequency coil and the matching network. Based on patient-specific parameters, the controller is configured to execute one or more rules to create a state of the matching network as well as the switched number of loops by turning individual coil loops on or off, and/or by making additional impedance adjustments using the matching network so that the total coil impedance can be matched to that of the preamplifier and the signal can be amplified by the preamplifier.

In general, the radio frequency antenna can be operated by the controller in different ways. According to a preferred embodiment of the invention, however, the radio frequency coil is provided with a switch which is controllable by the controller which enables the individual coil loops to be switched on and off, respectively, and a further switch which is controllable by the controller and which is arranged between the matching network and the preamplifier. This allows switching options to be established with low insertion loss.

Different coil loop configurations are possible. According to a preferred embodiment of the invention, however, the at least one further coil loop is formed as an additional serial or parallel connected winding in the first coil loop. By increasing the number of windings, the impedance of the input of the loop is higher and thus the losses in the matching network are reduced. By switching the number of loops and/or serial, parallel winding pattern the matching transformation ratio to the matching network and by that also to the preamplifier can be achieved.

In principle, different algorithms can be implemented in the controller. According to a preferred embodiment of the invention, however, a trained artificial neural network is implemented in the controller. Employing an artificial intelligence system such as an artificial neural network enables the dynamic matching with respect to the k-space amplitude. Even decision making of complex tasks, where threshold values and manually determined logic or rules for k-space applications may be not appropriate, can be realized. Furthermore, pre-scans can be omitted due to the output of the artificial neural network. The k-space is defined as an array of numbers representing spatial frequencies in the magnetic resonance image. The setting of the artificial neural network can depend on many parameters such as position and shape of the coil, the distance to tissue and the bore wall, the region of interest, the field strength and in general the employed type of coil.

The data for training the artificial neural network can be acquired with a prototype coil by scanning several phantoms anatomies with different matching network settings. The raw data is supposed to be the resulting images per coil and the artificial neural network settings. Further, using the data from the typically applied RF calibration procedures such as noise correlation matrix measurement and the frequency response curves. Furthermore, electromagnetic simulation techniques can be used to calculate sensitivity profiles that can be modified to resemble imaging data, which would eliminate the need for experimental trials.

These elements are input data that can be used in combination with output data generated by offline reconstruction to train an artificial neural network. With offline reconstruction, the output data is obtained by combining the images of the coils of an array, including the computation of the best fitting network setting. The trained artificial neural network can then generate the output data based on a complete or incomplete input data set.

The controller can be a field programmable gate array or some other processor, wherein due to the bandwidth limitation the field programmable gate array rule-based machine is directly in communication with the magnetic resonance imaging sequencer. In case of patient table movement, a start and stop interrupt is activatable, so that the dynamic matching process does not generate image artifacts.

In general, various patient-specific parameters can be used. According to a preferred embodiment of the invention, however, the patient-specific parameters comprise the distance and position of the radio frequency coil relative to the patient and the total coil impedance. These patient-specific parameters are determined while the patient is lying on the patient table in the bore directly before image acquisition.

It is possible to determine the total coil impedance in different ways. According to a preferred embodiment of the invention, however, the radio frequency coil has an impedance sensor with which the total coil impedance can be measured. Thereby the possibility is given to measure the individual total coil impedance.

In principle, various methods can be selected to determine the noise of the received signal. According to a preferred embodiment of the invention, however, the controller is adapted for determining the coil loops to be employed and for controlling the matching network by means of a noise measurement. This noise measurement takes place in the form of a pre-scan prior to image acquisition.

In general, it is possible to use an internal signal source for noise measurement. According to a preferred embodiment of the invention, however, an external signal source is provided for supporting the noise measurement. The external signal source is part of the system and galvanically isolated from the controller. Furthermore, the external signal source is known and remotely controllable and can thus tune the measurement system over the entire frequency range of interest. Because the signal source is separated from the radio frequency coil, the signal source can be placed directly next to it. This reduces the cable attenuation and improves the signal-to-noise ratio of the measurement.

It is possible to place the preamplifier outside the radio frequency coil. According to a preferred embodiment of the invention, however, the preamplifier is integrated in a radio frequency coil housing. This means that each radio frequency coil has a dedicated preamplifier.

In principle, the radio frequency coil can be analog. According to a preferred embodiment of the invention, however, the radio frequency coil is a digital coil and the controller is located in the radio frequency coil housing and a digital preamplifier is employed for signal amplification. Employing a digital coil enables digital signal acquisition and processing directly at the patient. The signal is digitized directly in the radio frequency coil nearest to the patient and is then transferred and processed throughout the imaging chain, which generally provides a better signal-to-noise ratio.
Further, according to the invention, a method of operating a radio frequency antenna system for a magnetic resonance imaging device is provided. The method comprises the following method steps: attaching an radio frequency coil to a patient which is to be examined by the magnetic resonance imaging device, wherein the radio frequency coil has a total coil impedance which is composed of a coil impedance and a patient-specific impedance and the radio frequency coil comprises a first switchable coil loop and at least one further switchable coil loop, analyzing patient-specific parameters, determining the coil loops to be switched on and off, respectively, and controlling a matching network based on the analyzed patient-specific parameters, receiving a signal by the switched coil loops of the radio frequency coil, impedance matching of the received signal by the matching network to the impedance of a preamplifier and amplifying the received signal by the preamplifier. Matching the impedance to the one of a preamplifier in this context means "approximating", i.e. it is intended to make the difference between the impedances small, preferably smaller than a predefined threshold value. Preferably, the radio frequency coil comprises multiple receive channels with their own amplifier and matching network, and the method is performed for all these receive channels.

According to a preferred embodiment of the invention, the method further comprises a trained artificial neural network installed on a controller which determines the coil loops to be switched on and off, respectively, and which controls the matching network. This is performed digitally, so that the user does not need to have any specific knowledge regarding the implementation.

According to a preferred embodiment of the invention a noise measurement is employed to determine the coil loops to be switched on and off, respectively, and to control the matching network.

Further, according to the invention, a computer program for noise impedance matching for a magnetic resonance imaging device, comprising instructions which, when the program is executed by a computer, cause the computer to perform a method according to any one of claims (first method claim to last method claim).

In general, the radio frequency can coil exclusively be used for signal reception. According to a preferred embodiment of the invention, however, the switch of the radio frequency coil is used as detune circuit. Depending on the configuration, it may be that a coil element is located off from the examination area and hardly contributes to the image. However, the preamplifier would still produce noise. In this case, it would be ideal to mismatch the radiofrequency coil and the preamplifier so that a noise-trap is created.

It is possible to use different types of switches. According to a preferred embodiment of the invention, however, the switches are Gallium nitride field-effect transistor (GaN-FET) or single pole double throw (SPDT) switches with low insertion loss. GaN-FET or SPDT switches have low switching losses compared to silicon field-effect transistor switches due to their fast switching capabilities.

There are several ways to detect the position of the radio frequency coil on the patient surface in the bore. According to a preferred embodiment of the invention, however, the radio frequency coil is provided with markings which are detectable by a camera. For this purpose, special infrared markers can be provided on the coil, which can be detected by an infrared-sensitive camera and do not distort the magnetic field.

In principle, rigid radio frequency coils can also be provided. According to a preferred embodiment of the invention, however, the radio frequency coil is designed to be flexible and stretchable. This allows the radiofrequency coil to be positioned as conformally as possible on the patient surface.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 schematically depicts a radio frequency antenna system according to a preferred embodiment of the invention;
Fig. 2 schematically depicts a second radio frequency antenna system according to a preferred embodiment of the invention;
Fig. 3 schematically depicts a third radio frequency antenna system according to a preferred embodiment of the invention;
Fig. 4 schematically depicts a fourth radio frequency antenna system according to a preferred embodiment of the invention; and
Fig. 5 schematically depicts a scheme of a method according to a preferred embodiment of the invention.

### DETAILED DESCRIPTION OF EMBODIMENTS

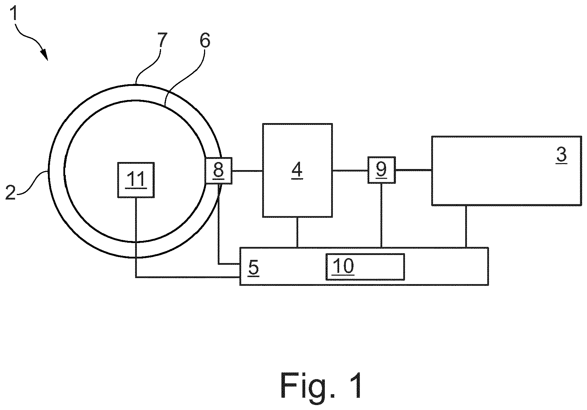

Fig. 1 schematically depicts a radio frequency antenna system 1 according to a preferred embodiment of the invention. The radio frequency antenna system 1 comprises a radio frequency coil 2, wherein the radio frequency coil 2 comprises a coil impedance and, when next to a patient in the bore of a magnetic resonance imaging device, a patient-specific impedance. A total coil impedance is composed of the coil impedance and the patient-specific impedance. A second part of the radio frequency antenna system 1 is the matching network 4. The matching network 4 is interconnected via a switch 8 with the radio frequency coil 2 and via a further switch 9 with a preamplifier 3. The switches 8,9 are Gallium nitride field-effect transistor (GaN-FET) switches with low insertion loss. The radio frequency coil 2 comprises a first coil loop 6 and a further coil loop 7. The first coil loop 6 and the further coil loop 7 are arranged parallel to each other and connected to the switch 8. The switch 8, which is controllable by means of a controller 5, enables the individual coil loops 6,7 to be switched on or off. The controller 5 is a field programmable gate array rule-based unit that contains a trained artificial neural network 10 and is connected to the matching network 4, the further switch 9, the preamplifier 3, and an impedance sensor 11 in addition to the switch 8. The impedance sensor 11 is also mounted within the bore of the magnetic resonance imaging device such that the measured impedance corresponds to the total coil impedance. This measured impedance is transferred as an input parameter to the trained artificial neural network 10, whereupon the trained neural network 10 determines the number of coil loops 6,7 switched into an on state, as well as the matching impedance adjustment to be made in the matching network 4, so that the noise impedance adjustment to the impedance of the preamplifier 3 is ensured, which in turn enables more advantageous signal transmission.

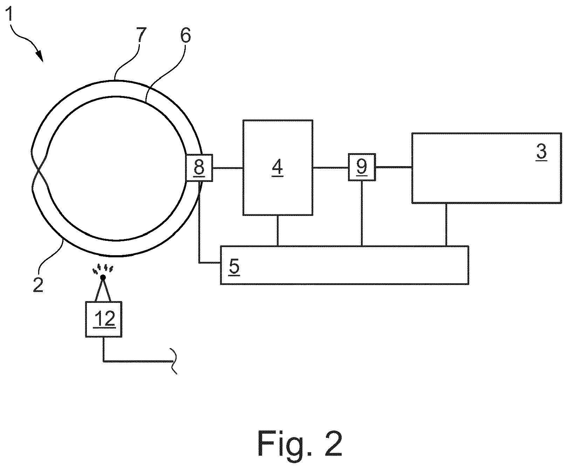

Fig. 2 schematically depicts a second radio frequency antenna system 1 according to a preferred embodiment of the invention. The further coil loop 7 is provided as a further winding arranged in series with the first coil loop 6, the controller 5 is adapted for determining the first coil loop 6 and the second coil loop 7 of the radio frequency coil 2 to be employed and for controlling the matching network 4 by means of a noise measurement. This noise measurement can be performed patient-specifically before the diagnostic imaging sequence or alternatively using phantoms as part of a quality assurance process, with phantoms serving as simulated patients. For this purpose, an external signal source 12 is provided, which is part of the radio frequency antenna system 1, but is galvanically separated from this system.

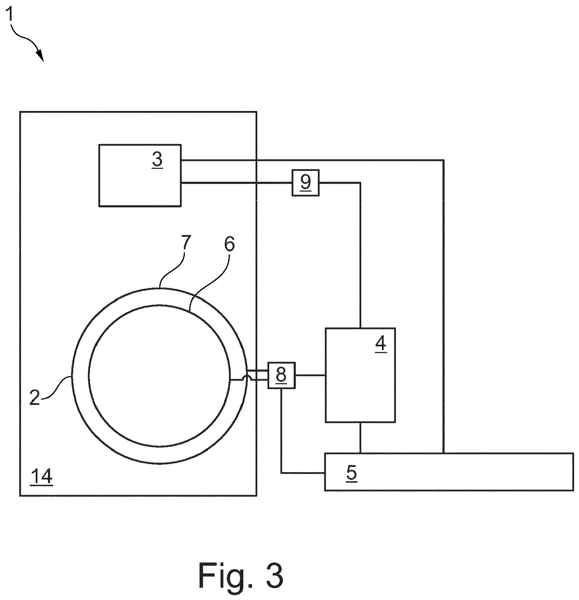

Fig. 3 schematically depicts a third radio frequency antenna system 1 according to a preferred embodiment of the invention, wherein the preamplifier 3 is integrated in a radio frequency coil housing 14 so that each radio frequency coil 2 has a dedicated preamplifier 3.

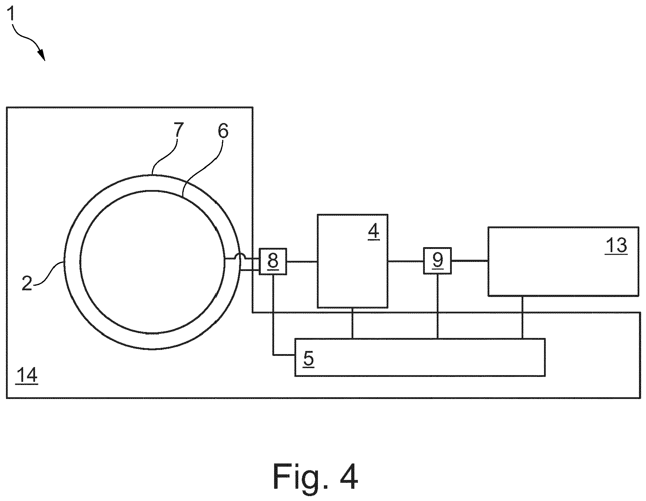

Fig. 4 schematically depicts a fourth radio frequency antenna system 1 according to a preferred embodiment of the invention wherein the radio frequency coil 2 is a digital coil and the controller 5 is located in the radio frequency coil housing 14 and a digital preamplifier 13 is employed for signal amplification.

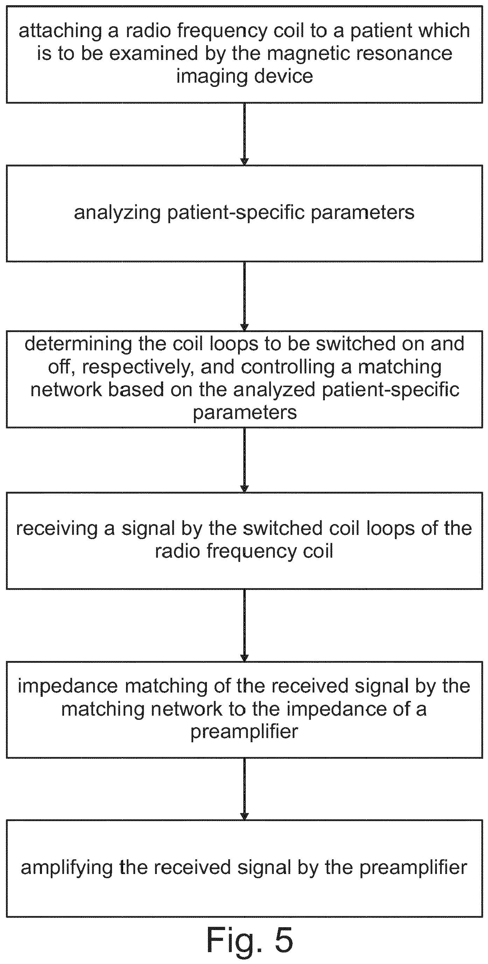

Fig. 5 schematically depicts a scheme of a method according to a preferred embodiment of the invention, wherein in a first step a radio frequency coil 2 is attached to a patient. The patient is to examined by the magnetic resonance imaging device. The radio frequency coil 2 has a total coil impedance which is composed of a coil impedance and a patient-specific impedance and the radio frequency coil 2 comprises a first switchable coil loop 6 and at least one further switchable coil loop 7.

Second, patient-specific parameters are analyzed.

Third, the coil loops 6, 7 to be switched on or off are determined, further the control of a matching network 4 is performed on the basis of the analyzed patient-specific parameters to achieve a noise impedance matching to the preamplifier.
Fourth, a signal by the switched coil loops 6, 7 of the radio frequency coil 2 is received,
Fifth, an impedance matching of the received signal by the matching network 4 to the impedance of a preamplifier 6 is performed.

Sixth, the received signal is amplified by the preamplifier 3.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of clearness, not all elements in the drawings may have been supplied with reference signs.

**REFERENCE SYMBOL LIST**

| | |
|---|---|
| Radio frequency antenna system | 1 |
| Radio frequency coil | 2 |
| Preamplifier | 3 |
| Matching network | 4 |
| Controller | 5 |
| First coil loop | 6 |
| Further coil loop | 7 |
| Switch | 8 |
| Further switch | 9 |
| Artificial neural network | 10 |
| Impedance sensor | 11 |
| External signal source | 12 |
| Digital preamplifier | 13 |
| Radio frequency coil housing | 14 |

## Claims

1. Radio frequency antenna system (1) for a magnetic resonance imaging device comprising
a radio frequency coil (2) which, when attached to a patient has a total coil impedance composed of a coil impedance and a patient-specific impedance,
a preamplifier (3) connected to the radio frequency coil (2), by means of which a signal received by the radio frequency coil (2) can be amplified and an amplified output signal can be output,
a matching network (4) which is interconnected between the radio frequency coil (2) and the preamplifier (3) with which the total coil impedance of the radio frequency coil (2) is adjustable and
a controller (5), wherein
the radio frequency coil (2) comprises a first coil loop (6) and at least one further coil loop (7),
the controller (5) is connected to the radio frequency coil (2), the matching network (4) and the preamplifier (3) and
the controller (5) is adapted for noise impedance matching between the radio frequency coil (2) and the preamplifier (3) as a function of patient-specific parameters by controlling the employment of the first coil loop (6) and the at least one further coil loop (7) and by controlling the matching network (4).

2. Radio frequency antenna system (1) according to claim 1, wherein the radio frequency coil (2) is provided with a switch (8) which is controllable by the controller which enables the individual coil loops (6,7) to be switched on and off, respectively, and a further switch (9) which is controllable by the controller (5) and which is arranged between the matching network (4) and the preamplifier (3).

3. Radio frequency antenna system according to any of the preceding claims, wherein the at least one further coil loop (7) is formed as an additional serial or parallel connected winding in the first coil loop (6).

4. Radio frequency antenna system (1) according to any of the preceding claims, wherein a trained artificial neural network (10) is implemented in the controller (5).

5. Radio frequency antenna system (1) according to any of the preceding claims, wherein the patient-specific parameters comprise the distance and position of the radio frequency coil (2) relative to the patient and the total coil impedance.

6. Radio frequency antenna system (1) according to any of the preceding claims, wherein the radio frequency coil (2) has an impedance sensor (11) with which the total coil impedance can be measured.

7. Radio frequency antenna system (1) according to any of the claims 1 to 3 and 6, wherein the controller (5) is adapted for determining the coil loops (6,7) to be employed and for controlling the matching network (4) by means of a noise measurement.

8. Radio frequency antenna system (1) according to claim 7, wherein an external signal source (12) is provided for supporting the noise measurement.

9. Radio frequency antenna system (1) according to any of the preceding claims, wherein the preamplifier (3) is integrated in the radio frequency coil housing (14).

10. Radio frequency antenna system (1) according to any of the preceding claims, wherein the radio frequency coil (2) is a digital coil and the controller (5) is located in the radio frequency coil housing (14) and a digital preamplifier (13) is employed for signal amplification.

11. A method of operating a radio frequency antenna system (1) for a magnetic resonance imaging device, comprising the following method steps:
attaching a radio frequency coil (2) to a patient which is to be examined by the magnetic resonance imaging device, wherein the radio frequency coil (2) has a total coil impedance which is composed of a coil impedance and a patient-specific impedance and the radio frequency coil (2) comprises a first switchable coil loop (6) and at least one further switchable coil loop (7),
analyzing patient-specific parameters,
determining the coil loops (6,7) to be switched on and off, respectively, and controlling a matching network (4) based on the analyzed patient-specific parameters,
receiving a signal by the switched coil loops (6,7) of the radio frequency coil (2),
impedance matching of the received signal by the matching network (4) to the impedance of a preamplifier (3) and
amplifying the received signal by the preamplifier (3).

12. Method of operating a radio frequency antenna system (1) according to claim 11, wherein a trained artificial neural network (10) installed on a controller (5) which determines the coil loops (6,7) to be switched on and off, respectively, and which controls the matching network (4).

13. Method of operating a radio frequency antenna system (1) according to claim 11, wherein a noise measurement is employed to determine the coil loops (6,7) to be switched on and off, respectively, and to control the matching network (4).

14. A computer program for noise impedance matching for a magnetic resonance imaging device, comprising instructions which, when the program is executed by a computer, cause the computer to perform a method according to any one of claims 11 to 13.
